# FASCICULE DE BREVET EUROPEEN

(11) **EP 2 542 520 B1**
(45) Date de publication et mention de la délivrance du brevet: **29.08.2018**
(21) Numéro de dépôt: 11712956.9
(22) Date de dépôt: 03.03.2011
(51) Int. Cl.: C07C 67/08, C07C 67/58, C07C 67/03, C09D 9/00, C09K 3/00, C09D 7/20

(54) **PROCÉDÉ D'OBTENTION DE COMPOSITIONS DE BIOSOLVANTS PAR ESTÉRIFICATION ET COMPOSITIONS DE BIOSOLVANTS OBTENUES**
VERFAHREN ZUM ERHALTEN VON BIOLÖSUNGSMITTELZUSAMMENSETZUNGEN DURCH VERESTERUNG UND ERHALTENE BIOLÖSUNGSMITTELZUSAMMENSETZUNGEN
METHOD FOR OBTAINING BIOSOLVENT COMPOSITIONS BY ESTERIFICATION AND RESULTING BIOSOLVENT COMPOSITIONS

(30) Priorité: 04.03.2010 FR 1051570
(43) Date de publication de la demande: 09.01.2013
(73) Titulaire: Centre National de la Recherche Scientifique (C.N.R.S.), 75016 Paris (FR); Université Claude Bernard Lyon 1, 69622 Villeurbanne, Cedex (FR); Institut Vietnamien de Chimie Industrielle, Hanoï (VN)
(72) Inventeur: ESSAYEM, Nadine, 38540 Saint Just Chaleyssin (FR); SAPALY, Gilbert, 69009 Lyon (FR); VU, Thi Thu Ha, Hanoi (VN); NGUYEN, Thi Thu Trang, Hanoi (VN); NGUYEN, Thi Thuy Ha, Hanoi (VN)
(74) Mandataire: Lavoix
(86) Numéro de dépôt international: PCT/FR2011/050441
(87) Numéro de publication internationale: WO 2011/107712

(56) Documents cités:
- GB-A- 1 282 926
- US-A- 1 651 666
- US-A- 4 608 202
- US-A1- 2005 143 599
- US-B1- 6 284 720
- RATHIN DATTA, MICHAEL HENRY: "Lactic acid: recent advances in products, processes and technologies - a review", JOURNAL OF CHEMICAL TECHNOLOGY AND BIOTECHNOLOGY, vol. 81, 2 mai 2006 (2006-05-02), pages 1119-1129, XP002603807,

## Description

L'invention concerne un nouveau procédé de préparation de compositions de biosolvants, par estérification, en une étape à partir d'acides issus de la biomasse. La présente demande décrit également les compositions de biosolvants ainsi obtenues. L'invention concerne également un procédé de valorisation des acides carboxyliques issus de la biomasse de préférence les acides fermentaires.
Les solvants constituent une classe de substances largement utilisées dans de nombreux secteurs économiques où ils jouent des rôles divers. Ce sont des liquides capables de dissoudre, de diluer ou d'extraire d'autres composés sans engendrer de modifications chimiques. Cependant, les solvants traditionnels sont généralement des composés organiques volatils, nocifs pour la santé et pour l'environnement. Les règlementations environnementales incitent, de nos jours, à utiliser des solvants de substitution, notamment dans les applications industrielles. Dans ce contexte, de nouveaux solvants, appelés biosolvants, issus de matières premières renouvelables, non toxiques et biodégradables sont apparus sur le marché. Ils présentent l'avantage d'offrir, d'une part une alternative aux ressources fossiles, notamment pétrolières, et d'autre part un bilan environnemental positif.
Les principaux biosolvants entrant en jeu dans les formulations de nettoyage, de produits phytosanitaires, d'encres d'imprimerie, de peintures, de vernis ou de liants bitumeux sont des esters d'acides organiques fermentaires comme le lactate d'éthyle, les esters d'acides gras, l'éthanol, les dérivés terpéniques, les dérivés du glycérol ou de sucres. Parmi ces composés biosourcés, le lactate d'éthyle et les esters d'acide gras sont connus pour leurs propriétés solvant, utilisés seuls ou en mélanges. On connaît notamment de US 6,284,720 une composition de biosolvants comprenant de 40 à 70% en poids d'un ester d'acide lactique en C1-C4, de préférence le lactate d'éthyle, et de 1 à 30% en poids d'un ester C1-C4 d'acide gras en C16-C20 ayant un point de fusion inférieur à -10 °C, de préférence esters éthyliques (qui présentent l'avantage d'être 100% biosourcés, l'éthanol étant produit par fermentation de sucres alors que le méthanol est issu du pétrole) ou méthyliques d'acides gras d'une huile végétale. La composition peut en outre comprendre des additifs, surfactants... On connaît également de US 6,191,087 une composition de biosolvant comprenant de 10 à 60% en poids d'un ester d'acide gras en C16-C20 ayant un point de fusion inférieur à -10 °C, de 20 à 75% en poids d'un ester C1-C4 d'acide lactique, de 0 à 20% en poids d'un surfactant, de 0 à 20% en poids d'un épaississant et de 0 à 50% en poids d'un solvant organique. La publication de Datta et al (Journal of Chemical Technology and Biotechnology, 2006, 81, 1119-1129) décrit les différentes applications de l'acide lactique et de ses dérivés, notamment l'utilisation de lactate d'éthyle dans des compositions de biosolvants.

Les compositions de biosolvants sont obtenues par mélange du lactate d'éthyle avec les esters d'acides gras souhaités. Le lactate d'éthyle est produit par estérification de l'acide lactique avec l'éthanol. Cependant, un problème majeur de cette réaction est qu'elle est équilibrée. Il est donc nécessaire pour obtenir un rendement correct de déplacer l'équilibre. Ceci est notamment possible en utilisant un excès d'éthanol ou en soutirant en continu l'eau formée lors de la réaction. Les solutions actuellement mises en place pour résoudre ce problème majeur sont à l'origine d'une augmentation sensible du coût du lactate d'éthyle. Un autre problème est l'oligomérisation de l'acide lactique au cours de la réaction.

De nombreuses recherches ont été menées pour pallier ces problèmes et améliorer le rendement en lactate d'éthyle. On connaît notamment de GB1282926 un procédé de séparation d'acides comprenant l'estérification de ces acides, la séparation des esters obtenus et la récupération des acides correspondants. Le procédé décrit comprend les étapes suivantes : une solution aqueuse contenant de l'acide lactique est mélangée avec un alcool miscible dans l'eau. La solution résultante est extraite en la chauffant modérément avec un solvant organique non-miscible à l'eau. L'acide lactique contenu dans la phase aqueuse est transformé en ester d'acide lactique, lequel ester passe dans la phase organique. Cette phase organique est ensuite distillée pour récupérer le solvant non miscible. Le résidu est ensuite distillé pour récupérer l'ester de l'acide lactique sous une forme pure et est ensuite transformé en acide. Ce procédé permet donc d'extraire l'ester formé de la phase aqueuse ce qui déplace l'équilibre vers la formation de l'ester. Cependant, les solvants utilisés sont des solvants organiques non biologiques et pour certains toxiques (benzène, chloroforme...).
On connaît de US 1,651,666 un procédé de préparation d'ester par réaction entre un mélange d'alcool, un acide et un solvant de l'ester correspondant. Cependant, le solvant de l'ester est une huile dérivée du pétrole à haut point d'ébullition. Ce procédé ne permet pas en une seule étape d'obtenir une composition de biosolvant comprenant des esters.

On connaît également de US2005/0143599 l'emploi d'un solvant dérivé du pétrole pour estérifier et extraire un acide dilué dans l'eau. Ce procédé vise spécifiquement la séparation d'acides et ne permet pas la préparation d'un biosolvant comprenant des esters en une seule étape.

On connaît de US 5,723,639 un procédé de préparation de lactate d'éthyle par réaction du lactate d'ammonium avec l'éthanol mettant en oeuvre une membrane de pervaporation. Cette membrane de pervaporation permet de laisser passer l'eau et l'ammoniac formé mais ne laisse pas passer l'alcool et l'ester formés. On a ainsi une extraction en continue de l'eau. La publication de Budd et al (Ind.Eng.Chem.Res., 2004, 43, 1863-1867) décrit également l'utilisation de membrane de pervaporation pour la réaction d'estérification entre l'acide lactique et l'éthanol. Cependant, de telles membranes sont relativement fragiles et de coût élevé. Le procédé de préparation de lactate d'éthyle par cette méthode est donc peu économique.

On connaît de WO2004/052826 un procédé de préparation de lactate d'éthyle par estérification entre l'acide lactique et l'éthanol. Ce procédé est basé sur l'existence d'un azéotrope eau/éthanol. Au cours du procédé, un mélange gazeux eau-éthanol est extrait en continu, cet extrait est déshydraté et permet de récupérer un flux d'éthanol pouvant être recyclé. Une fois encore ce procédé nécessite des équipements et traitements supplémentaires pour déplacer l'équilibre, ce qui augmente le coût de production du lactate d'éthyle.

On connaît enfin de WO01/47860 un procédé en deux étapes de production de lactate d'éthyle. La première étape consiste en une transformation, avec élimination d'eau, d'une composition d'acide lactique en une composition oligomérique d'acide lactique. Cette première étape permet l'élimination de l'eau qui pourrait être formée. La deuxième étape consiste en une estérification de tout ou partie de l'acide lactique contenu, sous forme monomérique, dimérique, oligomérique ou polymérique, à l'aide d'un catalyseur de transestérification. Ce procédé présente l'inconvénient d'être réalisé en deux étapes ce qui augmente les temps de réaction ainsi que le coût du procédé.

Ainsi depuis le dépôt du brevet GB1282926, les procédés de synthèse de lactate d'éthyle par estérification se sont compliqués, amenant à des procédés en deux étapes ou à des procédés utilisant des équipements supplémentaires voire des équipements coûteux et sensibles (membrane de pervaporation).

Les compositions de biosolvants comprenant du lactate d'éthyle sont obtenues en 2 étapes, une première étape de formation du lactate d'éthyle et une deuxième étape de mélange de ce lactate avec des biosolvants. De tels procédés en deux étapes augmentent sensiblement le prix de revient de la composition finale, nécessitant de plus des étapes complémentaires, notamment de purification du lactate d'éthyle.

Il y a donc un besoin de trouver un procédé de préparation de biosolvant à base de lactate d'éthyle qui soit plus compétitif tant en termes de rejets environnementaux qu'en termes de coût et notamment de fournir un procédé de synthèse de compositions de biosolvants en une seule étape.
Un objectif est également de trouver un tel procédé qui soit applicable pour d'autres acides issus de la biomasse.
Un objectif de la présente invention est de fournir un procédé de synthèse de compositions biosolvants en une étape à partir d'acides organiques issus de la biomasse qui permette de répondre aux inconvénients mentionnés ci-dessus et notamment ne nécessitant ni l'emploi d'un excès d'alcool, ni l'extraction en continue de l'eau formée.
Un autre objectif de la présente invention est de fournir un procédé de valorisation de la biomasse, et notamment des acides issus de la biomasse, par exemple produits suite à un processus de fermentation, en une composition de biosolvants.

La présente invention concerne un procédé de synthèse d'une composition comprenant au moins un ester lactique, l'acide lactique étant issu de la biomasse, et au moins un biosolvant organique choisi parmi les esters méthylique ou éthylique d'acides gras comprenant de 12 à 22 atomes de carbone seuls ou en mélange, ledit procédé comprenant une réaction d'estérification entre au moins l'acide lactique et au moins un alcool choisi parmi l'éthanol, le méthanol, le propanol ou le butanol, seuls ou en mélange, en présence d'un catalyseur acide et du biosolvant organique, ledit biosolvant organique étant sélectif de l'ester formé par rapport à l'acide lactique, et notamment, non miscible à la solution alcoolique d'acides issus de la biomasse.
Ainsi, le procédé selon l'invention comprend le mélange d'au moins un acide issu de la biomasse, au moins un alcool et un biosolvant organique, l'acide et l'alcool réagissent pour former un ester qui va se solubiliser dans le biosolvant organique. Par séparation des deux phases, d'une part le mélange acide/alcool, d'autre part le mélange biosolvant organique/ester, il est possible d'obtenir le mélange biosolvant organique/ester correspondant à la composition selon l'invention.
On entend par biomasse l'ensemble de la matière organique (carbohydrate) d'origine végétale ou animale, mais aussi tout produit dérivé ou effluent liquide issu de sa transformation mécanique, thermochimique, enzymatique ou de procédés fermentaires, ainsi que de leurs mélanges.
Les acides peuvent être issus de la biomasse par des processus de fermentation (notamment des sucres), par exemple les acides lactique, citrique, succinique, lévulinique... ou par pressage de la biomasse, par exemple l'acide aconitique issu du pressage de la canne à sucre.
Avant la mise en oeuvre du procédé de l'invention, les acides peuvent être séparés de la biomasse par des méthodes connues de l'homme du métier.
Les acides peuvent être mis en oeuvre purifiés ou non purifiés. Ainsi les acides, notamment non purifiés, peuvent comprendre de l'eau, notamment en des quantités liées à leur processus de formation.

Les alcools peuvent également être mis en oeuvre purifiés ou non. Ainsi, les alcools peuvent comprendre de l'eau, notamment en des quantités liées à leur processus de formation.

Pour le procédé de l'invention, on entend par « biosolvant organique sélectif de l'ester ou des esters formés par rapport à l'acide ou aux acides de départ», un biosolvant organique qui solubilise l'ester ou les esters et qui est insoluble dans le mélange alcool/acide. Le biosolvant est donc non soluble au mélange alcool/acide formant ainsi un milieu biphasique. L'ester ou les esters formés sont bien plus solubles dans le biosolvant organique que dans le mélange alcool/acide permettant ainsi une extraction de cet (ces) ester(s) dans le biosolvant. L'ester formé a notamment une plus grande solubilité dans le biosolvant organique que dans le mélange alcool/acide de départ.
Le milieu biphasique permet avantageusement la solubilisation et l'extraction de cet (ces) ester(s) par transfert de phase.

La plus grande solubilité de l'ester ou des esters formés dans le biosolvant que dans le mélange alcool/acide permet de déplacer l'équilibre et de recueillir une phase organique suffisamment riche en ester pour qu'il ne soit pas nécessaire de recycler ou retraiter la phase aqueuse. L'homme du métier, de part ses connaissances générales, pourra déterminer si le biosolvant présente une insolubilité envers le mélange acide+alcool suffisante pour être utilisé dans le procédé de l'invention. Ainsi, tous les biosolvants non solubles à la solution alcoolique d'acide issu de la biomasse, formant un milieu réactionnel bi-phasique et capables de solubiliser l'ester ou les esters formés par réaction entre l'acide et l'alcool peuvent être utilisés pour le procédé de l'invention.

Un mélange biphasique va se former entre d'une part le mélange {alcool + acide issu de la biomasse} et le biosolvant organique d'autre part. Sans être lié par une quelconque théorie, il apparaît que l'alcool et l'acide réagissent pour donner un ester qui va être solubilisé, dès sa formation, dans le biosolvant organique. Cette solubilisation de l'ester formé entraîne un déplacement de la réaction en faveur de la formation de l'ester permettant ainsi une augmentation du rendement en ester.
La nature biphasique de ce mélange réactionnel est également avantageuse car elle permet de séparer les phases si besoin, par exemple par des méthodes bien connues de l'homme du métier, par exemple par décantation, et/ou par ajout d'eau au milieu qui permet l'élimination des acides et alcools résiduels, et d'obtenir une composition de biosolvants comprenant en mélange le ou les ester(s) formé(s) et le biosolvant organique. Cette composition est directement utilisable sans nécessiter d'étape supplémentaire, notamment de purification.
Le mélange alcool/acide peut également comprendre de l'eau.
Selon un mode de réalisation le biosolvant organique n'est pas l'acide pélargonique.
Les biosolvants organiques qui peuvent être utilisés sont choisis parmi les esters d'acides gras d'origine naturelle et les éthers d'alkyle de glycérol, seuls ou en mélange.
Par esters gras on entend des esters aliphatiques possédant une chaîne carbonée de 4 à 28 atomes de carbones. Selon le procédé de l'invention les esters gras sont choisis parmi les esters aliphatiques possédant une chaîne carbonée de 12 à 22 atomes de carbone. Ils peuvent être obtenus par des procédés connus comme par exemple la transestérification de matières grasses d'origine végétale ou animale par le méthanol ou l'éthanol (méthodes usuelles de synthèse du biodiesel).
Les esters d'acides gras d'origine naturelle sont de préférence des esters produits par transestérification par l'éthanol ou le méthanol d'huile végétale ou de graisse animale, de préférence des esters éthylique et méthylique d'acides gras, seuls ou en mélange, notamment des esters éthyliques ou méthyliques d'acides gras en C₁₂ à C₂₂.
Selon le procédé de l'invention, les esters d'acides gras sont de préférence choisis parmi les esters méthylique ou éthylique d'huile de colza, d'huile de palme, de jatrofa, d'huile d'olive, d'huile de sésame, d'huile d'arachide, d'huile de maïs, d'huile de pavot, d'huile de carthame, d'huile de soja, d'huile de tournesol, d'huiles végétales usagées ou de graisses animales, seuls ou en mélange.

Selon le procédé de l'invention, les esters d'acides gras sont de préférence choisis parmi les esters méthylique ou éthylique d'huile de colza, d'huile d'olive, d'huile de sésame, d'huile d'arachide, d'huile de maïs, d'huile de soja, d'huile de tournesol seuls ou en mélange.

Par éthers du glycérol sont décrits des éthers de glycérol à chaîne grasse. De préférence les éthers de glycérol sont choisis parmi les mono, di ou tri alkyl glycérol éthers, avec des chaînes alkyles, de préférence en C12-C22, saturées ou insaturées, seuls ou en mélange. On entend par, chaîne alkyle insaturée, une chaîne alkyle totalement ou partiellement insaturée.

La quantité de biosolvant organique utilisée dans le présent procédé doit être suffisante pour permettre une solubilisation/extraction du ou des ester(s) formé(s).

Les propriétés de la composition de biosolvant, obtenue ou susceptible d'être obtnue, par le présent procédé, dépendent de la proportion en ses différents constituants. Ainsi, la quantité de biosolvant organique est déterminée selon les propriétés souhaitées pour la composition. De préférence, le rapport massique biosolvant organique/mélange {alcool+acide issu de la biomasse} est supérieur ou égal à 1:1.

De préférence, l'acide issu de la biomasse est notamment un acide issu de la fermentation de la biomasse.

Il est également décrit un procédé mettant en oeuvre un acide carboxylique (monoacide, diacide ou triacide) issu de la biomasse (de préférence obtenu par voie fermentaire) choisi parmi l'acide lactique, l'acide lévulinique, l'acide béhénique, l'acide gadoléique, l'acide succinique, l'acide adipique, l'acide glutarique, l'acide citrique, l'acide aconitique, l'acide itaconique, seul ou en mélange.

Le procédé de la présente invention peut être réalisé indifféremment par catalyse acide homogène ou hétérogène.

Dans un mode de réalisation, le catalyseur est un catalyseur acide homogène. Dans un mode de réalisation particulier, le catalyseur acide homogène est choisi parmi les acides minéraux forts, de préférence l'acide sulfurique, l'acide méthanesulfonique, acide triflique, acide trifluoro-méthane sulfonique, l'acide chlorhydrique, seul ou en mélange.

On entend par acides forts des acides homogènes ou hétérogènes caractérisés par une valeur d'acidité de Hammett -Ho>3.
Dans un mode de réalisation, le catalyseur est un catalyseur acide hétérogène.

Dans un mode de réalisation le catalyseur acide hétérogène est choisi dans le groupe constitué par les résines acides échangeuses d'ions, de préférence les résines sulfoniques échangeuses d'ions, notamment celles dont la base est un copolymère macroréticulé de styrène et de divinylbenzène, de capacité en groupes fonctionnels variables (Amberlyst® 15, 35... commercialisés par Rohm et Hass). On peut aussi utililiser dans le procédé des resines échangeuses d'ions perfluorées de type résines Nafion® commercialisées par du Pont de Nemours.

Dans un autre mode de réalisation le catalyseur est choisi dans le groupe constitué par les hétéropolyacides supportés sur charbon, de préférence les hétéropolyacides de structure de Keggin H₃PW₁₂O₄₀, H₄SiW₁₂O₄₀, H₃PMo₁₂O₄₀, H₄SiMo₁₂O₄₀; des sels acides alcalins de ces hétéropolyacides de préférence de formule AxH₃-xPM₁₂O₄₀ avec A = Cs⁺, K⁺, Rb⁺, NH₄⁺ et M=W ou Mo ; ou de formule AxH₄-xSiM₁₂O₄₀ A = Cs⁺, K⁺, Rb⁺, NH₄⁺ et M=W ou Mo ; des catalyseurs à base de charbon sulfoné ; des catalyseurs à base de support oxyde (oxyde de zirconium, de titane, d'aluminium, de niobium) modifiés par des oxoanions (sulfate, tungstate, phosphates..) ; des catalyseurs de type zéolithes acides (H-Beta, H-ZSM5...), des silices-alumines ou autres oxydes mixtes acides, des argiles cationiques.

De préférence le catalyseur est choisi dans le groupe constitué par les hétéropolyacides supportés sur charbon, de préférence les hétéropolyacides de structure de Keggin H₃PW₁₂O₄₀, H₄SiW₁₂O₄₀, H₃PMo₁₂O₄₀, H₄SiMo₁₂O₄₀; des sels acides alcalins de ces hétéropolyacides de préférence de formule AxH₃-xPM₁₂O₄₀ avec A = Cs⁺, K⁺, Rb⁺, NH₄⁺ et M=W ou Mo ; ou de formule AxH₄-xSiM₁₂O₄₀ A = Cs⁺, K⁺, Rb⁺, NH₄⁺ et M=W ou Mo ; des catalyseurs à base de charbon sulfoné ; des catalyseurs à base de support oxyde (oxyde de zirconium, de titane, d'aluminium, de niobium) modifiés par des oxoanions (sulfate, tungstate, phosphates..) ; des catalyseurs de type zéolithes acides (H-Beta, H-ZSM5...).

L'un des avantages majeurs de l'utilisation de catalyseurs acides hétérogènes est leur élimination facile et rapide du milieu réactionnel. En effet, une simple filtration suffira à éliminer le catalyseur du produit obtenu.

Le catalyseur, homogène ou hétérogène, est présent dans une quantité suffisante, notamment comprise entre 0,1 et 10% en poids par rapport à l'acide ou au mélange d'acides issus de la biomasse, de préférence entre 0,1 à 5% en poids, plus préférentiellement environ 1% en poids.

De préférence, l'alcool est choisi parmi éthanol, méthanol, propanol, butanol, seul ou en mélange.

Il n'y a a priori pas de limite supérieure concernant la quantité d'alcool utilisée dans le procédé. En effet, plus la quantité d'alcool est importante, plus la réactivité, et par conséquent le rendement en ester, sera importante. Cependant, la composition de biosolvant finale peut comprendre des traces d'alcool qui, si elles sont trop importantes, pourraient modifier ses propriétés. Ainsi, les propriétés souhaitées pour la composition de biosolvant finale peuvent être à l'origine d'une limitation de la quantité d'alcool utilisée.

Dans un mode de réalisation, le ratio alcool/acide est avantageusement compris entre 1 et 5, de préférence compris entre 2 et 5, par exemple il est d'environ 3.

Le procédé de la présente invention peut être réalisé à diverses températures. La vitesse de la réaction dépend de la température à laquelle elle est mise en oeuvre, ainsi plus la température est basse plus la réaction est lente. Au contraire, une augmentation de la température permet d'augmenter la vitesse de réaction.
La température de mise en oeuvre du procédé de la présente invention dépend, entre autre, de la nature des composés mis en jeu et notamment de leur stabilité thermique. L'homme du métier est à même de déterminer la température optimum à laquelle le procédé doit être réalisé.

De manière préférée le procédé est mis en oeuvre à la température d'ébullition du mélange réactionnel, la mise en oeuvre à une telle température représentant un bon compromis entre vitesse de réaction et conditions de réaction. Cependant, comme mentionné ci-dessus, le procédé peut être mis en oeuvre à plus faible température en contrepartie d'une diminution de la vitesse de réaction. Le procédé peut également être mis en oeuvre à une température supérieure à la température d'ébullition du mélange tout en restant à une température inférieure à la température limite de stabilité des catalyseurs.

Par exemple, lorsque le catalyseur est un catalyseur homogène, la température peut être comprise entre 40°C et 200°C.

Par exemple, lorsque le catalyseur est un catalyseur hétérogène, la température peut être comprise entre 40°C et la température maximale de stabilité du catalyseur.
Pour les Amberlyst®, température maximale de stabilité est de l'ordre de 130°C
Par exemple pour hétéropolyacides supportés sur charbon la température est de préférence comprise entre 40°C et 300°C.
De la même manière, le procédé peut être réalisé dans une gamme de pression variée. Dans un mode de réalisation, le procédé est réalisé à pression atmosphérique, sous un vide réduit, ou sous pression autogène du milieu. De préférence le procédé est réalisé sous pression atmosphérique.
Dans un mode de réalisation de l'invention, le procédé comprend en outre une étape de récupération de la composition de biosolvant formée. Dans un mode de réalisation cette étape de récupération est une décantation.
Dans un mode de réalisation de l'invention le procédé comprend en outre une étape d'élimination du catalyseur. Cette étape d'élimination du catalyseur peut être réalisée par toutes méthodes connues de l'homme du métier. Pour le cas des catalyseurs acides hétérogènes l'élimination du catalyseur peut être réalisée par simple filtration. Il est également décrit la composition de biosolvant obtenue par le procédé de la présente invention.
De manière avantageuse, la composition obtenue ou susceptible d'être obtenue par le procédé de l'invention peut notamment être utilisée en tant que produits de nettoyage d'encres d'imprimerie, dégraissage de pièces métalliques, dissolvants pour vernis à ongles, nettoyage de revêtements, dissolvants pour peintures, comme solvants dans les formulations d'encre d'imprimerie, formulations de pesticides et adjuvants phytosanitaires, produits de nettoyage ménager ou industriels, dans des produits cosmétiques, liants bitumineux, formulations d'esters « siccatifs ».
La présente invention concerne également un procédé de valorisation des acides carboxyliques issus de la biomasse par par estérification mettant en oeuvre le procédé de la présente invention pour obtenir des compositions de biosolvants.

Les exemples suivants permettent d'illustrer le procédé de l'invention et ses avantages. Ceux-ci étant donnés à titre d'illustration sans pour autant être limitatifs.

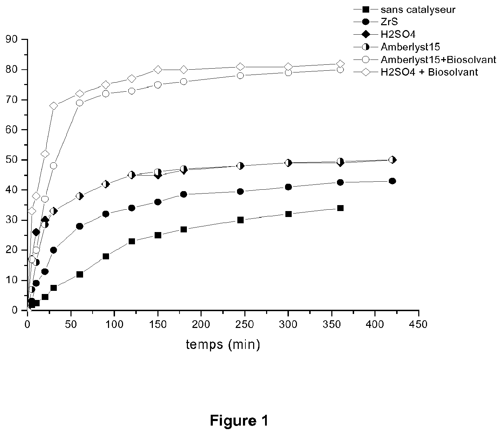

La figure 1 représente le rendement en lactate d'éthyle, en fonction du temps, de la réaction d'estérification entre l'acide lactique et l'éthanol, sans catalyseur ni biosolvant (a), en présence en tant que catalyseur de zircone sulfatée et sans biosolvant (b) en présence d'acide sulfurique en tant que catalyseur et sans biosolvant (c), en présence d'Amberlyst-15® en tant que catalyseur et sans biosolvant (d), en présence d'Amberlyst-15® en tant que catalyseur et de biosolvant (e), en présence d'acide sulfurique en tant que catalyseur et de biosolvant (f).

### Exemples comparatifs :

Les exemples ont été effectués avec de l'acide lactique en présence d'éthanol. Les expérimentations ont été menées avec différents catalyseurs ou en l'absence de catalyseur et en l'absence de biosolvant organique.

### Exemple a : Procédé réalisé en l'absence de catalyseur et de biosolvant organique

La synthèse de lactate d'éthyle est réalisée dans un ballon en pyrex équipé d'un réfrigérant. Les quantités suivantes de réactifs sont introduites dans le réacteur : 66,7g d'éthanol, 39,1g d'acide lactique (AL) (rapport molaire EtOH/AL = 3,3). Le milieu réactionnel est agité au moyen d'un agitateur magnétique. Le milieu réactionnel est porté à la température de réaction au moyen d'un bain d'huile régulé à 80°C. L'avancement de la réaction est suivi par prises d'échantillons. Le rendement molaire en lactate d'éthyle est déterminé par analyse chromatographie en phase gazeuse (GC).

Le rendement en lactate d'éthyle en fonction de la durée de la réaction est représentée figure 1, courbe a.

### Exemple b : Procédé réalisé en présence de zircone sulfatée en tant que catalyseur et en l'absence de biosolvant organique

La synthèse de lactate d'éthyle est réalisée dans un ballon en pyrex équipé d'un réfrigérant. Les quantités suivantes de réactifs sont introduites dans le réacteur : 66,7g d'éthanol, 39,1g d'acide lactique (rapport molaire EtOH/AL = 3,3). 1,2 g de zircone sulfatée (0,75 mmole H⁺), préalablement déshydratée une nuit dans une étuve à 110°C, est ajouté aux réactifs. Le milieu réactionnel est agité au moyen d'un agitateur magnétique. Le milieu réactionnel est porté à la température de réaction au moyen d'un bain d'huile régulé à 80°C. L'avancement de la réaction est suivi par prises d'échantillons. Le rendement molaire en lactate d'éthyle est déterminé par analyse GC.

Le rendement en lactate d'éthyle en fonction de la durée de la réaction est représentée figure 1, courbe b.

### Exemple c : Procédé réalisé en présence d'acide sulfurique en tant que catalyseur et en l'absence de biosolvant organique

La synthèse de lactate d'éthyle est réalisée dans un ballon en pyrex équipé d'un réfrigérant. Les quantités suivantes de réactifs sont introduites dans le réacteur : 66,7g d'éthanol, 39,1g d'acide lactique (rapport molaire EtOH/AL = 3,3). 0,33 g d'acide sulfurique 96% (6.46 mmole H⁺), est ajouté aux réactifs. Le milieu réactionnel est agité au moyen d'un agitateur magnétique. Le milieu réactionnel est porté à la température de réaction au moyen d'un bain d'huile régulé à 80°C. L'avancement de la réaction est suivi par prises d'échantillons. Le rendement molaire en lactate d'éthyle est déterminé par analyse GC.

Le rendement en lactate d'éthyle en fonction de la durée de la réaction est représentée figure 1, courbe c.

### Exemple d : Procédé réalisé en présence d'Amberlyst-15® en tant que catalyseur et en l'absence de biosolvant organique

La synthèse de lactate d'éthyle est réalisée dans un ballon en pyrex équipé d'un réfrigérant. Les quantités suivantes de réactifs sont introduites dans le réacteur : 66,7g d'éthanol, 39,1g d'acide lactique (rapport molaire EtOH/AL = 3,3). 1,4 g d'Amberlyst-15® (6,6 mmole H⁺), préalablement déshydratée une nuit dans une étuve à 110°C, est ajouté aux réactifs. Le milieu réactionnel est agité au moyen d'un agitateur magnétique. Le milieu réactionnel est porté à la température de réaction au moyen d'un bain d'huile régulé à 80°C. L'avancement de la réaction est suivi par prises d'échantillons. Le rendement molaire en lactate d'éthyle est déterminé par analyse GC.
Le rendement en lactate d'éthyle en fonction de la durée de la réaction est représentée figure 1, courbe d.

### Exemples de procédé selon l'invention :

Les exemples qui suivent ont été réalisés avec de l'acide lactique et de l'éthanol en présence d'un biosolvant, conformément à la présente invention.

### Exemple d' : Préparation du biosovant : ester méthylique d'huile de Colza

Les reactifs suivants sont mélangés dans un ballon en pyrex : 330g d'huile de Colza, 120g de MeOH, 3g de KOH. L'agitation est assurée au moyen d'un agitateur mécanique. Le ballon, équipé d'un réfrigérant est placé dans un bain d'huile régulé à 80°C. La réaction est stoppée après 2h30, l'ester méthylique est séparé de la glycérine par décantation. Analyse par GC de la phase ester méthylique pour vérifier que la transformation est totale.

### Exemple e : Procédé réalisé en présence d'Amberlyst-15® en tant que catalyseur et d'un biosolvant organique.

La synthèse de lactate d'éthyle est réalisée dans un ballon en pyrex équipé d'un réfrigérant. Les quantités suivantes de réactifs sont introduites dans le réacteur : 66,7g d'éthanol, 39,1g d'acide lactique (rapport molaire EtOH/AL = 3,3). 34g d'esters méthyliques d'huile de Colza obtenu selon l'exemple d' (biosolvant) sont introduits dans le ballon dans un ballon en pyrex. 1.4 g d'Amberlyst-15® (6,6 mmole H⁺), préalablement déshydratée une nuit dans une étuve à 110°C, est ajouté aux réactifs. Le milieu réactionnel est agité au moyen d'un agitateur magnétique. Le milieu réactionnel est porté à la température de réaction au moyen d'un bain d'huile régulé à 80°C. L'avancement de la réaction est suivi par prises d'échantillons. Le rendement molaire en lactate d'éthyle est déterminé par analyse GC.

Le rendement en lactate d'éthyle en fonction de la durée de la réaction est représentée figure 1, courbe e.

### Exemple f : Procédé réalisé en présence d'acide sulfurique en tant que catalyseur et d'un biosolvant organique.

La synthèse de lactate d'éthyle est réalisée dans un ballon en pyrex équipé d'un réfrigérant. Les quantités suivantes de réactifs sont introduites dans le réacteur : 66,7g d'éthanol, 39,1g d'acide lactique (rapport molaire EtOH/AL = 3,3). 34g d'esters méthyliques d'huile de Colza obtenus selon l'exemple d' (biosolvant) sont introduits. 0,33 g d'H₂SO₄ 96% (6,46 moles H⁺) sont ajoutés aux réactifs. Le milieu réactionnel est agité au moyen d'un agitateur magnétique. Le milieu réactionnel est porté à la température de réaction au moyen d'un bain d'huile régulé à 80°C. L'avancement de la réaction est suivi par prises d'échantillons. Le rendement molaire en lactate d'éthyle est déterminé par analyse GC.

Le rendement en lactate d'éthyle en fonction de la durée de la réaction est représentée figure 1, courbe f.

### Etude des résultats obtenus par les différents procédés

La figure 1, regroupant les résultats des différents exemples, montrent bien que l'utilisation de catalyseur permet d'augmenter le rendement en lactate d'éthyle lorsque le biosolvant est utilisé.

La figure 1 montre également l'influence de l'utilisation du biosolvant et du catalyseur dans le procédé. On note ainsi une augmentation de rendement, au bout de 350 minutes, de 30% (comparaison des courbes c et d et e et f).

Il est intéressant de noter que la nature du catalyseur, homogène (de type acide sulfurique) ou hétérogène (de type Amberlyst-15®), n'influe pas sur le rendement obtenu (comparaison des courbes e et f).

Enfin, la figure 1 montre que la vitesse d'apparition de lactate d'éthyle est beaucoup plus importante en présence de catalyseur et de biosolvant.

## Revendications

1. Procédé de synthèse d'une composition comprenant au moins un ester d'acide lactique, l'acide lactique étant issu de la biomasse et un biosolvant organique choisi parmi les esters méthylique ou éthylique d'acides gras comprenant de 12 à 22 atomes de carbone seuls ou en mélange, et comprenant la réaction d'estérification entre au moins l'acide lactique , et au moins un alcool choisi parmi l'éthanol, le méthanol, le propanol ou le butanol, seuls ou en mélange, en présence d'un catalyseur acide et du biosolvant organique, ledit biosolvant organique étant sélectif de l'ester formé par rapport à l'acide lactique en solubilisant l'ester, et non miscible à la solution alcoolique d'acides issus de la biomasse.

2. Procédé selon la revendication 1, pour lequel l'ester formé présente une plus grande solubilité dans le biosolvant organique que dans le mélange alcool/acide lactique.

3. Procédé selon l'une quelconque des revendications précédentes, pour lequel le mélange alcool/acide comprend de l'eau.

4. Procédé selon l'une quelconque des revendications 1 à 3, pour lequel le biosolvant est choisi parmi les esters méthylique ou éthylique issus d'huile de colza, d'huile de palme, d'huile de jatrofa, d'huile d'olive, d'huile de sésame, d'huile d'arachide, d'huile de maïs, d'huile de graines de pavot, d'huile de carthame, d'huile de soja, d'huile de tournesol, d'huiles végétales usagées ou de graisses animales seuls ou en mélange.

5. Procédé selon l'une quelconque des revendications 1 à 4, pour lequel le biosolvant est choisi parmi les esters méthylique ou éthylique issus d'huile de colza, d'huile d'olive, d'huile de sésame, d'huile d'arachide, d'huile de maïs, d'huile de soja ou d'huile de tournesol seuls ou en mélange.

6. Procédé selon l'une quelconque des revendications précédentes, pour lequel le catalyseur est un catalyseur acide homogène ou un catalyseur acide hétérogène.

7. Procédé selon la revendication 6, pour lequel le catalyseur est une résine sulfonique.

8. Procédé selon l'une quelconque des revendications précédentes, pour lequel le ratio en poids entre l'alcool et l'acide est compris entre 1 et 5.

9. Procédé selon l'une quelconque des revendications précédentes, pour lequel la quantité de catalyseur est comprise entre 0,1 et 10% en poids par rapport à l'acide lactique.

10. Procédé selon l'une quelconque des revendications précédentes, pour lequel le rapport massique biosolvant organique/mélange {alcool+acide issu de la biomasse} est supérieur ou égal à 1:1.

## Patentansprüche

1. Verfahren zum Synthetisieren einer Zusammensetzung umfassend mindestens einen Milchsäureester, wobei die Milchsäure aus der Biomasse ist, und ein organisches Biolösungsmittel ausgewählt aus Methyl oder Ethyl-Fettsäureestern mit 12 bis 22 Kohlenstoffatomen, allein oder im Gemisch, wobei das Verfahren die Veresterungsreaktion zwischen mindestens einer Milchsäure und mindestens einem Alkohol umfasst, wobei das Alkohol ausgewählt ist aus Ethanol, Methanol, Propanol oder Butanol, allein oder im Gemisch, in Anwesenheit eines säuerlichen Katalysators und des organischen Biolösungsmittels, wobei das organische Biolösungsmittel beim Auflösen des Esters für den gebildeten Ester gegenüber der Milchsäure selektiv ist, und nicht mischbar mit der alkoholischen Lösung von Säuren aus der Biomasse.

2. Verfahren nach Anspruch 1, wobei der gebildete Ester eine höhere Löslichkeit in dem organischen Biolösungsmittel als in der Alkohol/Milchsäure-Mischung aufweist.

3. Verfahren nach einem der vorhergehenden Ansprüche, wobei die Alkohol/Milchsäure-Mischung Wasser aufweist.

4. Verfahren nach einem der Ansprüche 1 bis 3, wobei das Biolösungsmittel ausgewählt ist aus Methyl oder Ethylestern aus Rapsöl, Palmöl, Jatrophaöl, Olivenöl, Sesamöl, Erdnussöl, Maisöl, Mohnöl, Distelöl, Sojaöl, Sonnenblumenöl, gebrauchtem Öl oder tierisches Fett, allein oder im Gemisch.

5. Verfahren nach einem der Ansprüche 1 bis 4, wobei das Biolösungsmittel ausgewählt ist aus Methyl oder Ethylestern aus Rapsöl, Olivenöl, Sesamöl, Erdnussöl, Maisöl, Sojaöl oder Sonnenblumenöl, allein oder im Gemisch.

6. Verfahren nach einem der vorhergehenden Ansprüche, wobei der Katalysator ein säuerlicher, homogener Katalysator oder ein säuerlicher, heterogener Katalysator ist.

7. Verfahren nach Anspruch 6, wobei der Katalysator ein Sulfonharz ist.

8. Verfahren nach einem der vorhergehenden Ansprüche, wobei das Gewichtsverhältnis zwischen dem Alkohol und der Säure zwischen 1 und 5 liegt.

9. Verfahren nach einem der vorhergehenden Ansprüche, wobei die Menge von Katalysator zwischen 0,1 und 10 Gewichts-% in Bezug auf der Milchsäure liegt.

10. Verfahren nach einem der vorhergehenden Ansprüche, wobei das Gewichtsverhältnis organisches Biolösungsmittel / Gemisch (Alkohol + Säure aus der Biomasse) größer als oder gleich 1:1 ist.

## Claims

1. A method for synthesizing a composition comprising at least one ester of lactic acid, the lactic acid stemming from biomass and an organic biosolvent chosen among methyl and ethyl esters of fatty acids comprising from 12 to 22 carbon atoms alone or in mixture, and comprising the esterification reaction between at least the lactic acid and at least one alcohol selected among ethanol, methanol, propanol or butanol, alone or in mixture, in the presence of an acid catalyst and of the organic biosolvent, said organic biosolvent being selective of the ester formed relatively to lactic acid, and non-miscible with the alcoholic solution of acid stemming from the biomass.

2. The method according to claim 1, for which the ester formed has greater solubility in the organic biosolvent than in the alcohol/lactic acid.

3. The method according to any of the preceding claims, for which the alcohol/acid mixture comprises water.

4. The method according to any of claims 1 to 3, for which the organic biosolvent is selected from methyl or ethyl esters stemming from rape seed oil, palm oil, jatrofa oil, olive oil, sesame oil, ground nut oil, maize oil, poppy seed oil, safflower oil, soya bean oil, sunflower oil, used vegetable oils or animal oils, alone or as a mixture.

5. The method according to any of claims 1 to 4, for which the biosolvent is selected from methyl or ethyl esters stemming from rape seed oil, olive oil, sesame oil, ground nut oil, maize oil, soya bean oil, sunflower oil alone or as a mixture.

6. The method according to any of the preceding claims, for which the catalyst is a homogeneous acid catalyst or a heterogeneous acid catalyst.

7. The method according to claim 12, for which the catalyst is a sulfonic resin.

8. The method according to any of the preceding claims, for which the weight ratio between the alcohol and the acid is comprised between 1 and 5.

9. The method according to any of the preceding claims, for which the amount of catalyst is comprised between 0.1 and 10% by weight based on lactic acid.

10. The method according to any of the preceding claims, for which the organic biosolvent/{alcohol+acid stemming from the biomass} mixture mass ratio is greater than or equal to 1:1.
The present invention relates to a method for synthesizing a composition comprising at least one acid ester stemming from the biomass and an organic biosolvent, said method comprising the esterification reaction between at least one acid stemming from the biomass and at least one alcohol, in the presence of an acid catalyst and of the organic biosolvent, said organic biosolvent being selective of the ester or of the esters formed relatively to the acid or to the starting acids and non-miscible with the alcoholic solution of the acid stemming from the biomass.
